# EUROPÄISCHE PATENTANMELDUNG

(11) **EP 1 048 286 A2**
(43) Veröffentlichungstag der Anmeldung: **02.11.2000**
(21) Anmeldenummer: 00106487.2
(22) Anmeldetag: 25.03.2000
(51) Int. Cl.: A61K 7/06

(54) **Haarbehandlungsmittel mit Polymeren aus ungesättigten Sacchariden, ungesättigten Saccharidsäuren oder deren Derivaten**

(30) Priorität: 30.04.1999 DE 19919785
(71) Anmelder: Wella Aktiengesellschaft, 64274 Darmstadt (DE)
(72) Erfinder: Allwohn, Jürgen, Dr., 65558 Burgschwalbach (DE); Birkel, Susanne, Dr., 64380 Rossdorf (DE)

(57) **Zusammenfassung**

Es werden Haarbehandlungsmittel beschrieben mit einem Gehalt an mindestens einem Polymer oder Copolymer, welches aus mindestens einer Monomerart aufgebaut ist, welche ausgewählt ist aus
(a) ethylenisch ungesättigten Sacchariden oder deren Derivaten und
(b) ethylenisch ungesättigten Saccharidsäuren oder deren Derivaten
in einer geeigneten kosmetischen Grundlage, wobei die ungesättigten Saccharide und Saccharidsäuren in cyclischer oder offenkettiger Form vorliegen können.

## Beschreibung

Gegenstand der vorliegenden Erfindung ist ein Haarbehandlungsmittel mit einem Gehalt an mindestens einem Polymer oder Copolymer aus ungesättigten Sacchariden, ungesättigten Saccharidsäuren oder deren Derivaten.

Ein ansprechendes äußeres Erscheinungsbild wurde schon immer als sehr wichtig angesehen. Eine besondere Rolle spielt dabei die Frisur. Basis für ein ansprechendes Äußeres ist gut frisiertes und gepflegtes Haar. Zu den Forderungen, die an gut frisiertes Haar gestellt werden, zählt beispielsweise, daß das Haar im Trockenen einen angenehmen Griff, Elastizität, Volumen, Glanz und Halt haben sollte. Für die Reinigung und Pflege des Haares gibt es eine ganze Reihe von Haarbehandlungsmitteln wie Shampoos, Kuren, Spülungen, Spitzenfluids, die in den unterschiedlichsten Anwendungsarten, zum Beispiel als leave on- oder als rinse off-Produkte, appliziert werden. Neben diesen Pflegeprodukten kommen drei weitere Produktkategorien bei der Haarveränderung zum Einsatz, nämlich permanente oder temporäre Haarfärbemittel, permanente Haarverformungsmittel in Form von mildalkalischen oder sauren Dauerwellen beziehungsweise Haarentkrausungsmitteln sowie Mittel, die nur eine temporäre Verformung und Stabilisierung der Frisur ermöglichen und allgemein als Stylingmittel bekannt sind. Hierzu zählen Produkte wie Haarsprays, Haarlacke, Festigerlotionen, Festigerschäume, Haargele, glanzgebende Produkte, Frisurcremes etc.. Allen diesen Mitteln ist gemeinsam, daß sie in der Regel aus einer Vielzahl an Einzelsubstanzen oder Komponenten bestehen, die die unterschiedlichsten Aufgaben innerhalb der Rezeptur erfüllen.

Von besonderer Bedeutung sind dabei Polymere, die je nach Konstitution haarpflegende und/oder haarfestigende Eigenschaften besitzen, die Produktkonsistenz positiv beeinflussen, den Transport und die Haftung von Wirkstoffen am Haar verbessern oder die Eigenschaften der übrigen Inhaltsstoffe vorteilhaft modifizieren. Von besonderem Vorteil sind Polymere, die auf der Basis von nachwachsenden Rohstoffen beruhen und die gut biologisch abbaubar sind. Wünschenswert ist insbesondere ein zusätzlicher Schutzeffekt, z.B. ein Schutz der Haare vor Austrocknen bzw. eine Regulation des Feuchtigkeitshaushaltes der Haare durch ein verbessertes Wasserrückhaltevermögen von die Haare umhüllenden Polymeren. Die Aufgabe dieser Erfindung bestand also darin, neue Polymere zu finden, die eine oder mehrere dieser Funktionen erfüllen.

Eine solche Klasse von Polymeren wurde mit den im folgenden näher erläuterten Polymeren aus ungesättigten Sacchariden, Saccharidsäuren oder deren Derivaten gefunden. Diese sind in einer Vielzahl von verschiedenen Haarbehandlungsmitteln vorteilhaft einsetzbar.

Gegenstand der Erfindung ist daher ein Haarbehandlungsmittel mit einem Gehalt an mindestens einem Polymer oder Copolymer, welches aus mindestens einer Monomerart aufgebaut ist, welche ausgewählt ist aus
(a) ethylenisch ungesättigten Sacchariden oder deren Derivaten und
(b) ethylenisch ungesättigten Saccharidsäuren oder deren Derivaten,
in einer geeigneten kosmetischen Grundlage, wobei die ungesättigten Saccharide und Saccharidsäuren in cyclischer oder offenkettiger Form vorliegen können. Es können auch Mischungen der Polymere oder Copolymere eingesetzt werden. Als Derivate werden insbesondere solche Verbindungen angesehen, in denen die Säuregruppen als Salze, Ester, Lactone, Amide, Imide oder Nitrile vorliegen bzw. in denen die Hydroxylgruppen in alkylierter oder acylierter Form vorliegen oder mit einer üblichen Schutzgruppe versehen sind sowie Verbindungen, die unter eine der unten genannten allgemeinen Formeln I bis XVII fallen. Zu den Derivaten zählen auch die aus Hexitolen oder Pentitolen durch Wasserabspaltung abgeleiteten Hexenitole oder Pentenitole, welche in Form cyclischer Ether oder in linearer Form vorliegen können, wobei die Hydroxylgruppen mit üblichen Schutzgruppen versehen sein können und wobei eine endständige Carbonsäuregruppe oder eine Ketogruppe im Molekül enthalten sein kann. Das erfindungsgemäße kosmetische Mittel enthält bevorzugt 0,01 bis 25 Gewichtsprozent, besonders bevorzugt 0,05 bis 10 Gewichtsprozent der erfindungsgemäßen, von Sacchariden abgeleiteten Polymere oder Copolymere.

Die Polymere sind herstellbar durch radikalische Polymerisation von ethylenisch ungesättigten Mono-, Di- oder Oligosacchariden, Hexenitolen, Pentenitolen oder deren Derivaten, welche mindestens eine ethylenische Doppelbindung im Ring (endocyclisch), am Ring (exocyclisch) oder in der offenkettigen Form enthalten. Die ungesättigten Saccharide oder Saccharidsäuren können chemisch geschützt oder ungeschützt, oder enzymatisch oder chemisch modifiziert vorliegen.

Geeignete Polymere und Copolymere sowie deren Herstellung werden in der WO 95/25135 und in der WO 99/00436 beschrieben. Die dort beschriebenen Polymere und Copolymere sind Bestandteil dieser Anmeldung. Geeignete, von ungesättigten Sacchariden abgeleitete Monomere sind solche der allgemeinen Formeln I bis X:

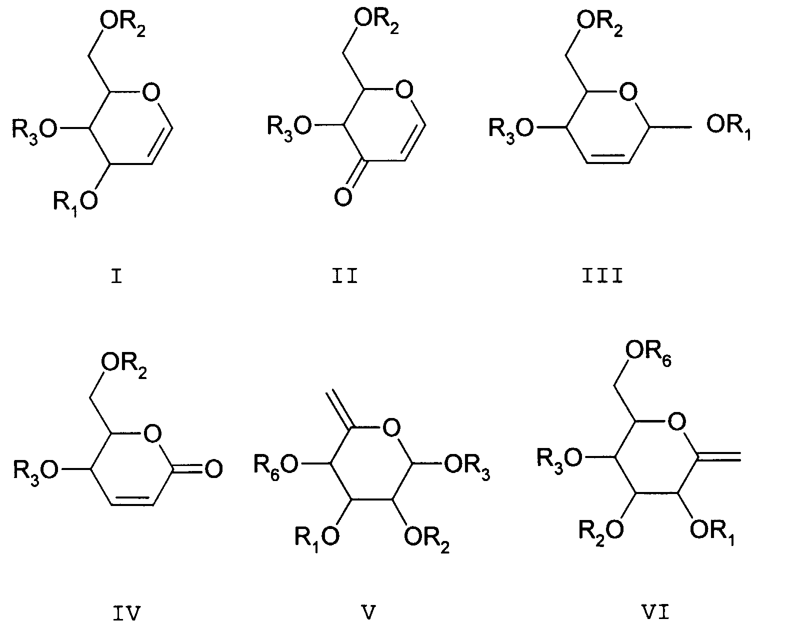

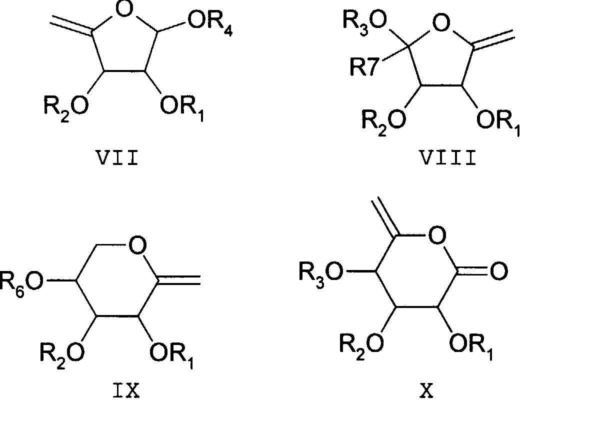

Geeignete, von ungesättigten Saccharidsäuren abgeleitete Monomere sind solche der allgemeinen Formeln XI bis XVII: 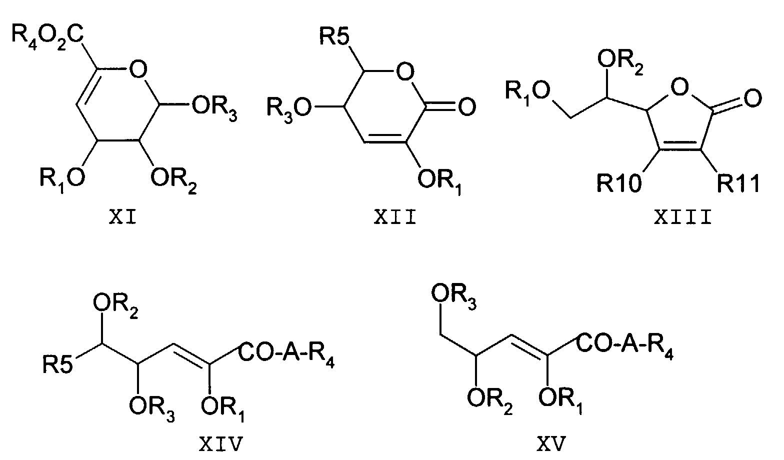 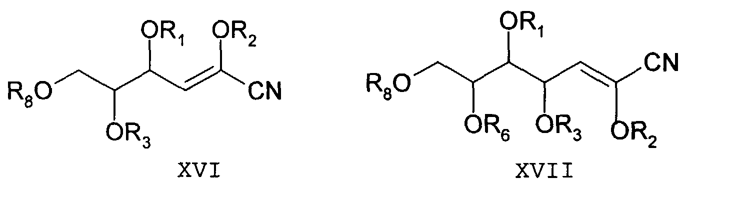 wobei R1, R2, R6 und R8 unabhängig voneinander für Wasserstoff, Alkyl, Aralkyl, Acyl oder Silyl stehen oder je zwei der genannten Reste bei geeigneter räumlicher Konfiguration Teil einer gemeinsamen Alkylidengruppe sind; R3 für einen der vorgenannten Reste oder für Glycosyl steht; R4 und R9 für Wasserstoff, Alkyl oder ein geeignetes Gegenion stehen; R5 für Wasserstoff, CH₂OR8 oder COOR9 steht; R7 für Wasserstoff oder O-Glycosyl steht; R10 und R11 unabhängig voneinander für Hydroxyl, Acetamido, O-Acyl oder O-Alkyl stehen und A für Sauerstoff, NH oder N-Alkyl steht. Die genannten Alkylgruppen enthalten vorzugsweise 1 bis 24, besonders bevorzugt 1 bis 6 Kohlenstoffatome.

Die Saccharid- und Saccharidsäuremonomere oder deren Derivate können mit einem oder mehreren radikalisch polymerisierbaren, nicht von Sacchariden abgeleiteten Comonomeren, insbesondere Vinylmonomeren copolymerisiert sein. Die Comonomere können hydrophil oder hydrophop, nichtionisch, kationisch, anionisch, zwitterionisch oder amphoter sein. Durch geeignete Wahl von Art und Mengen der Comonomere können die Löslichkeits-, Filmbildungs-, Haarfestigungs- und Haarpflegeeigenschaften der resultierenden Copolymere gezielt eingestellt werden. Vorzugsweise werden die hydrophilen, von Sacchariden abgeleiteten Monomere mit hydrophoben Co-Monomeren kombiniert.

Geeignete nichtionische Comonomere sind beispielsweise Acrylamid, Methacrylamid, Alkyl- und Dialkylacrylamid, Alkyl- und Dialkylmethacrylamid, ethylenisch ungesättigte Ester von C3- bis C10-Carbonsäuren (z.B. Alkylacrylat oder Alkylmethacrylat), Maleinsäuredialkylester, Vinylcaprolacton, fünf- bis achtgliedrige N-Vinyllactame, welche am Ring durch bis zu 3 C1- bis C12-Alkylreste substituiert sein können, insbesondere Vinylpyrrolidon und Vinylcaprolactam, weiterhin Vinylalkohol und Vinylester (z.B. Vinylacetat oder Vinylpropionat), Styrol, welches am aromatischen Ring durch ein oder zwei C1- bis C3-Alkylreste substituiert sein kann. Die Alkylgruppen dieser Monomere sind vorzugsweise C1- bis C7-Alkylgruppen, besonders bevorzugt C1-bis C3-Alkylgruppen. Weitere geeignete nichtionische copolymerisierbare Einheiten sind Silikonmakromere mit einer endständigen ethylenisch ungesättigten Gruppe, wie sie beispielsweise in der EP 0 412 704 beschrieben werden. Die ungesättigten Silikonmacromere besitzen die allgemeine Formel X-(Y)ₙ-Si(R)₃₋ₘ(Z)ₘ, wobei X eine Vinylgruppe, Y eine divalente Verbindungsgruppe, R Wasserstoff, Alkyl, Aryl oder Alkoxy und Z eine monovalente Polysiloxangruppe mit einem Molekulargewicht von mindestens 500 ist und n für 0 oder 1 und m für 1 bis 3 steht. Die genannten Alkylgruppen enthalten vorzugsweise 1 bis 24, besonders bevorzugt 1 bis 6 Kohlenstoffatome.

Als anionische Comonomere werden auch solche Monomere bezeichnet, welche durch Neutralisation einer Säuregruppe anionisierbar sind. Geeignete anionische Comonomere sind beispielsweise monoethylenisch ungesättigte C3- bis C10-Carbonsäuren und deren Alkalimetall-, Erdalkalimetall- oder Ammoniumsalze wie z.B. Acrylsäure, Methacrylsäure, Dimethylacrylsäure, Ethylacrylsäure, Vinylessigsäure, Allylessigsäure, Vinylpropionsäure, Crotonsäure, Maleinsäure bzw. Maleinsäureanhydrid oder Maleinsäuremonoester.

Als kationische Comonomere werden auch solche Monomere bezeichnet, welche durch Protonierung von basischen Stickstoffen kationisierbar sind. Geeignete kationische Comonomere sind beispielsweise Monomere, welche kationische oder kationisierbare Gruppen, vorzugsweise primäre, sekundäre, tertiäre oder quaternäre Stickstoffgruppen enthalten. Geeignete ammoniumsubstituierte Vinylmonomere sind zum Beispiel Trialkylmethacryloxyalkylammonium, Trialkylacryloxyalkylammonium, Dialkyldiallylammonium, quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, welches am heterocyclischen Ring mit bis zu 3 C1- bis C12-Alkylresten substituiert sein kann, Alkylvinylpyridinium, oder Alkylvinylpyrrolidon Salze. Geeignete aminsubstituierte Vinylmonomere sind zum Beispiel Dialkylaminoalkylacrylat, Dialkylaminoalkylmethacrylat, Monoalkylaminoalkylacrylat und Monoalkylaminoalkylmethacrylat, N-Vinylimidazol, welches am Ring mit bis zu 3 C1- bis C12-Alkylresten substituiert sein kann. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Zwitterionische oder amphotere Comonomere enthalten sowohl basische oder kationische Gruppen als auch saure oder anionische Gruppen. Geeignete Monomere mit zwitterionischen Gruppen enthalten die funktionelle Gruppe -NR1R2⁺-A-COO⁻, wobei R1 und R2 unabhängig voneinander Alkylgruppen mit 1 bis 24, vorzugsweise 1 bis 4 C-Atomen und A eine divalente Verbindungsgruppe, beispielsweise eine Alkylengruppe mit 1 bis 24, vorzugsweise 1 bis 4 C-Atomen bedeuten, wobei die Reste R1 und R2 so verbunden sein können, daß sie einen das N-Atom enthaltenden Ring bilden, welcher vorzugsweise 6-gliedrig ist. Ein bevorzugtes Monomer ist Methacryloylethylbetain.

Die erfindungsgemäß einzusetzenden Polymere oder Copolymere können weiterhin über freie Hydroxylgruppen mit Polyethylenglykol- oder Polypropylenglykolketten verknüpft sein. Die unter Verwendung von ungesättigten Lactonen erhältlichen Polymere können durch Hydrolyse der Lactongruppe in saure bzw. nach Neutralisation in anionische Polymere überführt werden.

Die erfindungsgemäß einzusetzenden Saccharidpolymere zeichnen sich durch eine hohe Affinität zum Haar aus. Sie legen sich an das Haar an und verstärken dadurch den Halt der Frisur. Setzt man die Saccharidpolymere in typischen Haarpflegemitteln ein wie Lotionen, Schäumen, Flüssig- oder Schaumfestigern, so erhält damit behandeltes Haar im Trockenen Zustand gute Festigung und Spannkraft bei gleichzeitig natürlichem Griff. Die Saccharidpolymere zeichnen sich weiterhin durch ein hohes wasserdampfaufnahmevermögen und durch ein hohes Feuchtigkeitsrückhaltevermögen aus. Sie können daher zur Regulierung des Feuchtigkeitshaushaltes des Haares oder zum Schutz der Haare vor Austrocknen vorteilhaft verwendet werden. Bei Kombination der Saccharidpolymere mit üblichen filmbildenden Polymeren wird die verbesserte Wassserdampfaufnahme und das verbesserte Feuchtigkeitsrückhaltevermögen auch für die aus der Kombination aufgebauten Filme beobachtet. Dies ist insbesondere vorteilhaft für Kombinationen mit Polymeren, die, wenn alleine eingesetzt, harte und spröde Filme bilden und keinerlei oder nur sehr geringe Wasserdampfaufnahme und Feuchtigkeitsspeicherung zeigen.

Ein weiterer Gegenstand der Erfindung ist daher ein Haarbehandlungsmittel mit einem Gehalt an
(A) mindestens einem von Sacchariden bzw. Saccharidsäuren oder deren Derivaten abgeleiteten Polymer oder Copolymer im Sinne dieser Anmeldung und
(B) mindestens einem filmbildenden Polymer in einer geeigneten kosmetischen Grundlage.

Die filmbildenden Polymere (B) liegen vorzugsweise in einer Menge von 0,01 bis 25, besonders bevorzugt von 0,1 bis 20 Gewichtsprozent vor und können einzeln oder in einem Gemisch eingesetzt werden. Unter filmbildenden Polymeren sollen solche Polymere verstanden werden, die in der Lage sind, auf Haaren einen Polymerfilm abzuscheiden. Die filmbildenden Polymere können nichtionisch, kationisch, anionisch, zwitterionisch oder amphoter sein und synthetischen oder natürlichen Ursprungs sein.

Geeignete anionische Polymere sind synthetische Homo- oder Copolymere mit neutralisierbare Säuregruppen enthaltenden Monomereinheiten, welche gegebenenfalls mit Comonomeren, die keine Säuregruppen enthalten, copolymerisiert sind. Als Säuregruppen kommen Sulfonsäure-, Phosphorsäure- und Carbonsäuregruppen in Betracht, von denen die Carbonsäuregruppen bevorzugt sind. Geeignete Säuregruppen enthaltende Monomere sind beispielsweise Acrylsäure, Methacrylsäure, Crotonsäure, Maleinsäure bzw. Maleinsäureanhydrid, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes anionisches, natürliches Polymer ist beispielsweise teilweise oder vollständig neutralisierter Schellack.

Bevorzugte Polymere mit Säuregruppen sind vernetzte oder unvernetzte Vinylacetat/Crotonsäure Copolymere, die beispielsweise in Form einer 60%igen Lösung in Isopropanol/Wasser unter der Handeisbezeichnung ARISTOFLEX® von der Firma HOECHST/Deutschland beziehungsweise von der Firma BASF unter dem Handelsnamen LUVISET® CA-66 vertrieben werden. Weitere geeignete anionische Polymere sind zum Beispiel Terpolymere aus Vinylacetat, Crotonsäure und Polyethylenoxid sowie Terpolymere aus Acrylsäure, Alkylacrylat und N-Alkylacrylamid, insbesondere Acrylsäure/Ethylacrylat/N-t-Butylacrylamid Terpolymere, wie sie unter den Handelsnamen ULTRAHOLD® 8 und ULTRAHOLD® STRONG der Firma BASF/Deutschland vertrieben werden oder Terpolymere aus Vinylacetat, Crotonat und Vinylalkanoat, insbesondere Vinylacetat/Crotonat/Vinylneodecanoat Copolymere, wie sie z.B. von der Firma National Starch unter der Handelsbezeichnung RESYN 28-2930 vertrieben werden.

Eine weitere Klasse von geeigneten anionischen Polymeren sind anionische Polyurethane. Bevorzugte Polyurethane sind dadurch gekennzeichnet, daß sie (a) endständige Säuregruppen besitzen, die beispielweise über Aminosulfonsäuren oder Aminocarbonsäuren eingeführt wurden, (b) gegebenenfalls weitere freie Carbonsäuregruppen enthalten, die durch Einpolymerisieren von Carbonsäurediolen wie beispielsweise Dimethylolpropansäure als Comonomere eingeführt wurden und (c) Polyurethansequenzen enthalten, die aus Polyesterdiolen und Diisocyanaten wie beispielsweise Alkylendiisocyanaten oder Isophorondiisocyanat gebildet wurden. Geeignet ist beispielsweise Luviset® PUR der Firma BASF/Deutschland.

Die anionischen Polymere liegen im erfindungsgemässen Mittel teilweise oder vollständig mit einem kosmetisch verträglichen Neutralisationsmittel neutralisiert vor. Als Neutralisationsmittel können organische oder anorganische Basen verwendet werden. Beispiele für Basen sind insbesondere Aminoalkanole wie z.B. Aminomethylpropanol (AMP), Triethanolamin oder Monoethanolamin, aber auch Ammoniak, NaOH u.a..

Geeignete synthetische, nichtionische, filmbildende, haarfestigende Polymere sind zum Beispiel Homo- oder Copolymere, welche aus mindestens einem nichtionischen Monomer aufgebaut sind. Nichtionische Monomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, wobei die Alkylgruppen dieser Monomere vorzugsweise C1- bis C7-Alkylgruppen, besonders bevorzugt C1-bis C3-Alkylgruppen sind. Geeignete synthetische, nichtionische, filmbildende, haarfestigende Polymere sind zum Beispiel Homopolymere des Vinylpyrrolidons sowie Homopolymere des N-Vinylformamids. Weitere geeignete synthetische filmbildende, nichtionische haarfestigende Polymere sind zum Beispiel Copolymerisate aus Vinylpyrrolidon und Vinylacetat, Terpolymere aus Vinylpyrrolidon, Vinylacetat und Vinylpropionat, Polyacrylamide, Polyvinylalkohole oder Polyethylenglykole mit einem Molekulargewicht von 800 bis 20.000 g/mol.

Geeignete natürliche filmbildende Polymere sind zum Beispiel Chitosan mit einem Molekulargewicht von 20.000 bis ca. 5 Millionen g/mol, wie es beispielsweise von der Firma Pronova vertrieben wird, oder verschiedene Saccharidtypen wie zum Beispiel Polysaccharide oder Gemische aus Oligo-, Mono- und Disacchariden, welche beispielsweise unter dem Handelsnamen C-PUR® von der Firma Cerestar, Brüssel/Belgien vertrieben werden. Weitere geeignete, natürliche Polymere sind chinesiches Balsamharz und Cellulosederivate, zum Beispiel Hydroxypropylcellulose mit einem Molekulargewicht von 30.000 bis 50.000 g/mol.

Geeignete filmbildende kationische Polymere sind dadurch gekennzeichnet, daß sie aus mindestens einer Monomerart aufgebaut sind, die kationische oder kationisierbare Gruppen, vorzugsweise primäre, sekundäre, tertiäre oder quaternäre Stickstoffgruppen enthält. Geeignete ammoniumsubstituierte Vinylmonomere sind zum Beispiel Trialkylmethacryloxyalkylammonium, Trialkylacryloxyalkylammonium, Dialkyldiallylammonium, quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, welches am heterocyclischen Ring mit bis zu 3 C1- bis C12-Alkylresten substituiert sein kann, Alkylvinylpyridinium, oder Alkylvinylpyrrolidon Salze. Geeignete aminsubstituierte Vinylmonomere sind zum Beispiel Dialkylaminoalkylacrylat, Dialkylaminoalkylmethacrylat, Monoalkylaminoalkylacrylat und Monoalkylaminoalkylmethacrylat, N-Vinylimidazol, welches am Ring mit bis zu 3 C1- bis C12-Alkylresten substituiert sein kann. Die Alkylgruppen dieser Monomere sind vorzugsweise niedere Alkylgruppen wie zum Beispiel C1- bis C7-Alkylgruppen, besonders bevorzugt C1- bis C3-Alkylgruppen. Die kationischen bzw. basischen Monomere können mit nicht-kationischen bzw. nicht-basischen Comonomeren copolymerisiert sein.

Geeignete kationische Polymere sind zum Beispiel Polyvinylpyrrolidon/Dimethylaminoethylmethacrylat Copolymer, ein Copolymer aus Polyvinylpyrolidon und Imidazoliminmethochlorid, ein Terpolymer aus Dimethyldiallylammoniumchlorid, Natriumacrylat und Acrylamid, ein Terpolymer aus Vinylpyrrolidon, Dimethylaminoethylmethacrylat und Vinylcaprolactam, mit quaternierten Ammoniumgruppen substituierte Hydroxyethylcellulose, Vinylpyrrolidon/Methacrylamidopropyltrimethylammoniumchlorid Copolymer oder diquaternäre Polydimethylsiloxane (INCI: Quaternium-80).

Geeignete amphotere Polymere sind Polymere, welche sowohl kationische oder durch Protonierung kationisierbare Gruppen als auch anionische oder durch Deprotonierung anionisierbare Gruppen enthalten. Kationische Gruppen sind beispielsweise quaternäre Amingruppen, kationisierbare Gruppen sind beispielsweise primäre, sekundäre oder tertiäre Amingruppen. Anionische Gruppen sind beispielsweise Carboxylat-, Sulfat-, Sulfonat-, Phosphat- oder Phosphonatgruppen. Anionisierbare Gruppen sind beispielsweise die protonierten Formen der genannten anionischen Gruppen.

Geeignete amphotere Polymere sind z.B. Copolymere aus Octylacrylamid, t-Butylaminoethylmethacrylat und zwei oder mehr Monomeren, bestehend aus Acrylsäure, Methacrylsäure oder deren Estern. Weitere Beispiele sind Copolymere von Acrylsäure, Methylacrylat und Methacrylamidopropyltrimethylammoniumchlorid (INCI: Polyquaternium-47), Copolymere aus Acrylamidopropyltrimoniumchlorid und Acrylaten oder Copolymere aus Acrylamid, Acrylamidopropyltrimoniumchlorid, 2-Amidopropylacrylamidsulfonat und DMAPA (INCI: Polyquaternium-43).

Das erfindungsgemäße Mittel kann darüber hinaus weitere, für Haarbehandlungsmittel übliche Zusatzbestandteile enthalten, zum Beispiel kationische Kämmbarkeitsverbesserer, Öle, Silikonöle, Verdicker und anderes mehr. Ebenso können Konsistenzgeber, wie sie üblicherweise für Cremes eingesetzt werden, enthalten sein, beispielsweise Fettalkohole, Fettalkoholsulfate oder Fettalkoholethersulfate. Ebenfalls enthalten sein können bei Raumtemperatur flüssige, wachsartige oder feste Polyethylenglykole. Selbstverständlich kann das erfindungsgemäße Mittel auch weitere übliche kosmetische Zusätze, wie verdickende Polymere sowie deren Kombination in einer Menge von vorzugsweise 0,01 bis 15 Gewichtsprozent; Parfümöle in einer Menge von vorzugsweise 0,01 bis 5 Gewichtsprozent; Trübungsmittel wie z.B. Ethylenglykoldistearat, Styrol/PVP Copolymere oder Polystyrole in einer Menge von vorzugsweise 0,01 bis 5 Gewichtsprozent; Netzmittel, Tenside oder Emulgatoren mit oder ohne Waschaktivität aus den Klassen der nichtionischen, anionischen, kationischen oder amphoteren oberflächenaktiven Substanzen wie Fettalkoholsulfate, ethoxylierte Fettalkohole, Fettsäurealkanolamide wie zum Beispiel die Ester der hydrierten Rizinusölfettsäuren in einer Menge von vorzugsweise 0,1 bis 20 Gewichtsprozent; ferner Feuchthaltemittel, Farbstoffe, Lichtschutzmittel, Antioxidantien, Glanzgeber und Konservierungsstoffe in einer Menge von vorzugsweise 0,01 - 10 Gewichtsprozent enthalten. Als weitere Zusätze sind geeignete wasserlösliche oder wasserunlösliche Silikonpolymere in einer Konzentration von 0,01 bis 50 Gewichtsprozent, bevorzugt in einer Konzentration von 0,1 bis 5 Gewichtsprozent einsetzbar.

Das erfindungsgemäße Haarbehandlungsmittel kann in verschiedenen Applikationsformen Anwendung finden, z.B. in Form von Haarpflege-, Haarfestigungs-, Haarreinigungs-, Haarfärbe- oder Tönungsmittel, als Blondiermittel oder als Dauerweilmittel. Das Mittel kann als Lotion, Schaum, Milch, Gel, Creme, Gelschaum, Aerosol- oder Nonaerosolspray, Haarwachs oder in Form eines emulsionsförmigen Haarpflegemittels (Haarspülung, Conditioner) appliziert werden. Das Mittel kann auch als Shampoo in Kombination mit anionischen, amphoteren, nichtionischen oder kationischen Tensiden oder als Färbemittel in Kombination mit direktziehenden oder oxidativen Haarfarben, z.B. als Farbfestiger vorliegen. Besonders bevorzugt ist die Anwendung als nicht auszuspülendes Leave-in-Produkt.

Wenn das erfindungsgemäße Haarbehandlungsmittel in Form eines Aerosolsprays vorliegt, so enthält es zusätzlich 15 bis 85 Gewichtsprozent, bevorzugt 25 bis 75 Gewichtsprozent eines Treibmittels und wird in einem Druckbehälter abgefüllt. Als Treibmittel sind beispielsweise niedere Alkane, wie zum Beispiel n-Butan, i-Butan und Propan, oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie beispielsweise N₂, N₂O und CO₂ sowie Gemische der vorstehend genannten Treibmittel geeignet.

Wenn das erfindungsgemäße Haarbehandlungsmittel in Form eines versprühbaren Non-Aerosol-Haarsprays vorliegt, so wird es mit Hilfe einer geeigneten mechanisch betriebenen Sprühvorrichtung versprüht. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Wenn das erfindungsgemäße Haarbehandlungsmittel in Form eines Haarschaumes (Mousse) vorliegt, so enthält es mindestens eine übliche, hierfür bekannte schaumgebende Substanz. Das Mittel wird mit oder ohne Hilfe von Treibgasen oder chemischen Treibmitteln verschäumt und als Schaum in das Haar eingearbeitet und ohne Ausspülen im Haar belassen. Das erfindungsgemäße Mittel weist als zusätzliche Komponente eine Vorrichtung zum Verschäumen der Zusammensetzung auf. Unter Vorrichtungen zum Verschäumenen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden.

Wenn das erfindungsgemäße Haarbehandlungsmittel in Form eines Haargels vorliegt, so enthält es zusätzlich mindestens eine gelbildende Substanz, beispielsweise eines der oben aufgeführten Verdickungsmittel in einer Menge von vorzugsweise 0,05 bis 10, besonders bevorzugt von 0,1 bis 2 Gew.%. Die Viskosität des Gels beträgt vorzugsweise von 500 bis 50.000 cSt, besonders bevorzugt von 1.000 bis 15.000 cSt bei 25°C (gemessen mit einem Rotationsviskosimeter nach DIN 53 018 T1 u. 2).

Wenn das erfindungsgemäße Haarbehandlungsmittel in Form eines Haarwachses vorliegt, so enthält es zusätzlich wasserunlösliche Fett- oder Wachsstoffe oder Stoffe, die der Zusammensetzung eine wachsähnliche Konsistenz verleihen, in einer Menge von vorzugsweise 0,5 bis 30 Gewichtsprozent. Geeignete wasserunlösliche Stoffe sind beispielsweise Emulgatoren mit einem HLB-Wert unterhalb von 7, Silikonöle, Silikonwachse, Wachse (z.B. Wachsalkohole, Wachssäuren, Wachsester, sowie insbesondere natürliche Wachse wie Bienenwachs, Carnaubawachs, etc.), Fettalkohole, Fettsäuren, Fettsäureester oder hochmolekulare Polyethylenglykole mit einem Molekulargewicht von 800 bis 20.000, vorzugsweise von 2.000 bis 10.000 g/mol.

Wenn das erfindungsgemäße Haarbehandlungsmittel in Form einer Haarlotion vorliegt, so liegt es als im wesentlichen nicht-viskose oder gering viskose, fließfähige Lösung, Dispersion oder Emulsion mit einem Gehalt an mindestens 10 Gewichtsprozent, vorzugsweise 20 bis 95 Gewichtsprozent eines kosmetisch verträglichen Alkohols vor. Als Alkohole können insbesondere die für kosmetische Zwecke üblicherweise verwendeten niederen Alkohole mit 1 bis 4 Kohlenstoffatomen wie zum Beispiel Ethanol und Isopropanol verwendet werden.

Wenn das erfindungsgemäße Haarbehandlungsmittel in Form einer Haarcreme vorliegt, so liegt es vorzugsweise als Emulsion vor und enthält entweder zusätzlich viskositätsgebende Inhaltsstoffe wie die oben genannten Verdicker in einer Menge von 0,1 bis 10 Gewichtsprozent oder die erforderliche Viskosität und cremige Konsistenz wird durch Micellbildung mit Hilfe von geeigneten Emulgatoren, Fettsäuren, Fettalkoholen, Wachsen etc. in üblicher Weise aufgebaut.

Wird das erfindungsgemäße kosmetische Mittel zur Haarpflege oder -festigung eingesetzt, so wird es folgendermaßen angewandt: Nach der Haarwäsche werden in dem handtuchtrockenen Haar, je nach Haarfülle, 5 bis 30 g des Mittels verteilt. Anschließend wird das Haar durchgekämmt und zur Frisur geformt und getrocknet.

Wenn das erfindungsgemäße Mittel als Mittel zur dauerhaften Verformung des Haares vorliegt, enthält es zusätzlich 0,5 bis 15 Gewichtsprozent mindestens einer keratinreduzierenden Mercaptoverbindung und liegt bevorzugt als wäßrige, alkalisch oder schwach sauer (pH 5 bis 10) eingestellte Zubereitung vor, welche als keratinreduzierende Mercaptoverbindung beispielsweise Cystein, Cysteamin, N-Acetyl-L-Cystein, Mercaptocarbonsäure, wie zum Beispiel Thioglykolsäure oder Thiomilchsäure, oder Salze von Mercaptocarbonsäuren, wie zum Beispiel Ammonium- und Guanidinsalze der Thioglykolsäure oder Thiomilchsäure enthält.

Wird das erfindungsgemäße Mittel in Form eines Haartönungsmittels eingesetzt, so enthält es zusätzlich 0,05 bis 2,0 Gewichtsprozent mindestens eines direkt auf das Haar aufziehenden Haarfarbstoffs, der beispielsweise aus den folgenden Klassen direkt auf das Haar aufziehender Haarfarbstoffe ausgewählt sein kann: aromatische Nitrofarbstoffe, zum Beispiel 1,4-Diamino-2-nitrobenzol, Azofarbstoffe, zum Beispiel Acid Brown 4 (C. I. 14 805), Anthrachinonfarbstoffe, zum Beispiel Disperse Voilet 4 (C. I. 61 105), Triphenylmethanfarbstoffe, zum Beispiel Basic Violet 1 (C. I. 42 535), wobei die Farbstoffe je nach Art ihrer Substituenten sauren, nichtionischen oder basischen Charakter haben können und/oder natürliche Haarfarbstoffe, wie zum Beispiel Henna oder Reng, der zur Farbentwicklung nicht der Oxidation bedarf.

Die nachfolgenden Beispiele sollen den Gegenstand der Erfindung näher erläutern.

### Beispiele

In den Beispielen wurden die folgenden, von ungesättigten Sacchariden abgeleiteten Polymere verwendet:
- Polymer (1):: Copolymer, erhalten aus Polymerisation von 3,4,5-Tri-O-benzoyl-1-deoxy-2,6-anhydro-D-fructose-hex-1-enitol und Maleinsäureanhydrid analog Beispiel 29 der WO 95/25135 und anschließender Hydrolyse der Benzoylgruppen und des Anhydrids
- Polymer (2):: Copolymer aus 2,4,6-Tri-O-acetyl-3-deoxy-D-erythro-hex-2-enono-1,5-lacton und Vinylacetat im Verhältnis 21:79, hergestellt analog Beispiel 5 der WO 99/00436
- Polymer (3):: Copolymer aus 2,4,6-Tri-O-acetyl-3-deoxy-D-erythro-hex-2-enono-1,5-lacton und Vinylpyrrolidon im Verhältnis 45:55, hergestellt analog Beispiel 5 der WO 99/00436
- Polymer (4):: Copolymer aus 2,4,6-Tri-O-acetyl-3-deoxy-D-erythro-hex-2-enono-1,5-lacton und Butylvinylether im Verhältnis 50:50, hergestellt analog Beispiel 5 der WO 99/00436

### Beispiel 1: Messung des Wasserdampfaufnahmevermögens

Es wurden fünf wäßrige Lösungen A bis E hergestellt mit einem Gehalt an den folgenden Polymeren:
- A:: 1 Gew.% Saccharidpolymer (1)
- B:: 5 Gew.% Octylacrylamide/Acrylates/Butylaminoethyl Methacrylate Copolymer (Amphomer®)
- C:: 5 Gew.% Amphomer® + 0,05 Gew.% Saccharidpolymer (1)
- D:: 5 Gew.% Vinylpyrrolidon/Vinylacetat Copolymer (Luviskol® VA 37)
- E:: 5 Gew% Luviskol® VA37 + 1 Gew.% Saccharidpolymer (1)

Für jede der Lösungen wurde eine Dreifachbestimmung durchgeführt. Es wurden Wägegläschen je zur Hälfte mit der zu untersuchenden Lösung befüllt. Bei 40°C im Umlufttrockenschrank wurde innerhalb von 24 Stunden das Lösungsmittel vollständig abgedampft und getrocknet. Nach Abkühlen wurde das Wägegläschen 48 Stunden bei 31% relativer Luftfeuchte und 22°C gehalten und anschließend die Einwaage bestimmt. Danach wurde das Wägegläschen 48 Stunden bei 71% relativer Luftfeuchte und 22°C gehalten und die Einwaage erneut bestimmt. Aus der Differenz der Wägungen wird die Wasserdampfaufnahme (WDA) berechnet.

**Tabelle 1**

| Feuchtigkeitsrückhaltevermögen (Restfeuchte) von Polymerfilmen | | |
|---|---|---|
| Polymer | WDA in % | Erwartungswert bei additivem Verhalten |
| A | 137.7 ± 0.2 | - |
| B | 6.2 ± 0.2 | - |
| C | 25.0 ± 0.2 | 7.4 |
| D | 3.4 ± 0.2 | - |
| E | 29.7 ± 0.1 | 4.8 |

Die erfindungsgemäßen Polymerkombinationen C und E zeigen ein erheblich größeres Wasserdampfaufnahmevermögen als aufgrund der Eigenschaften der Einzelsubstanzen zu erwarten ist.

### Beispiel 2: Haarbalsam

- 0,50 g: Saccharidpolymer (2)
- 6,00 g: Glycerylstearat/Polyethylenglykol-(20)-cetearylether
- 4,00 g: Diquaternäres Polydimethylsiloxan (Abil® Quat 3272)
- 2,00 g: Cetylalkohol
- 1,36 g: Zitronensäure
- 0,14 g: 1,2-Dibrom-2,4-dicyanobutan
- 0,12 g: Parfüm
- ad 100 g: Wasser

### Beispiel 3: Haarspülung

- 0,75 g: Saccharidpolymer (3)
- 4,00 g: Cetylstearylalkohol
- 1,36 g: DL-2-Pyrrolidon-5-carbonsäure
- 0,75 g: Cetyltrimethylammoniumchlorid
- 0,50 g: Parfüm
- 0,20 g: Pflanzenextrakt Extrapon® 5 Spezial der Firma Dragoco/Deutschland
- ad 100 g: Wasser

### Beispiel 4: Versprühbares Haarkurmittel

- 0,15 g: Saccharidpolymer (2)
- 2,00 g: Dimethyldiallylammoniumchlorid
- 1,25 g: Polyethylenglykol-(40)-sorbitanmonopalmitat
- 1,00 g: DL-2-Pyrrolidon-5-carbonsäure
- 0,10 g: Parfüm
- 0,03 g: Cetyltrimethylammoniumchlorid
- 15,15 g: Ethanol
- ad 100 g: Wasser

### Beispiel 5: Schaumförmiges Haarkurmittel

- 0,40 g: Saccharidpolymer (3)
- 2,00 g: Kationische Emulsion von aminfunktionalisiertem Polydimethylsiloxan (Dow Corning 929 Cationic Emulsion)
- 1,30 g: Zitronensäure
- 0,50 g: Hydroxypropylcellulose (M = 1.150.000 g/mol)
- 0,30 g: Silikonwachs Dow Corning 2501 Cosmetic Wax
- 0,20 g: Parfüm
- 0,25 g: Cetyltrimethylammoniumchlorid
- 0,15 g: D-Panthenol
- 0,10 g: Elastinhydrolysat
- 5,00 g: Propan/Butan (5,0 bar)
- 10,00 g: Ethanol
- ad 100 g: Wasser

### Beispiel 6: Haarfestigungsmittel

- 0,10 g: Saccharidpolymer (3)
- 3,00 g: Vinylpyrrolidon/Vinylacetat Copolymer
- 0,90 g: Ameisensäure
- 0,20 g: 1,2-Propylenglykol
- 0,15 g: Parfüm
- 0,03 g: Cetyltrimethylammoniumchlorid
- 20,20 g: Wasser
- ad 100 g: Ethanol

### Beispiel 7: Schaumfestiger

- 0,25 g: Saccharidpolymer (3)
- 3,15 g: Polyvinylpyrrolidon
- 1,60 g: Zitronensäure
- 0,60 g: Hydriertes Rizinusöl, ethoxyliert mit 40 Mol Ethylenoxid
- 0,22 g: Decylpolyglucosid
- 0,20 g: Vinylpyrrolidon/Methacrylamidopropyltrimethylammoniumchlorid Copolymer
- 0,20 g: Parfüm
- 6,00 g: Propan/Butan (5,0 bar)
- ad 100 g: Wasser

### Beispiel 8: Haarspray

- 0,05 g: Saccharidpolymer (3)
- 3,50 g: Vinylacetat/Crotonsäure/Vinylneodecanoat Terpolymer
- 0,15 g: Parfüm
- 0,14 g: Ameisensäure
- 45,00 g: Dimethylether
- ad 100 g: Ethanol

### Beispiel 9: 80% VOC-Haarspray

- 0,10 g: Saccharidpolymer (3)
- 0,10 g: Saccharidpolymer (2)
- 4,00 g: t-Butylacrylat/Ethylacrylat/Methacrylsäure Terpolymer
- 0,72 g: 2-Amino-2-methyl-1-propanol
- 0,20 g: Cyclo-Tetra(dimethylsiloxan)
- 0,05 g: Parfüm
- 15,00 g: Wasser
- 40,00 g: Dimethylether
- ad 100 g: Ethanol

### Beispiel 10: 55% VOC-Pumpspray

- 0,10 g: Saccharidpolymer (3)
- 3,50 g: Vinylacetat/Crotonsäure Copolymer
- 0,28 g: Ameisensäure
- 0,20 g: Parfüm
- 55,00g: Ethanol
- ad 100 g: Wasser

### Beispiel 11: Haargel

- 0,20 g: Saccharidpolymer (2)
- 2,50 g: Hydroxypropylmethylcellulose
- 0,80 g: Polyoxyethylen-(20)-sorbitanmonopalmitat
- 0,50 g: Polyoxyethylen-(25)-p-aminobenzoesäure
- 0,40 g: Ameisensäure
- 0,12 g: Cis-1-(3-Chlorallyl)-3,5,7-triaza-1-azoniaadamantan-chlorid
- 0,10 g: Parfüm
- 23,00 g: Glycerin (86prozentig)
- ad 100 g: Wasser

### Beispiel 12: Farbfestiger

- 0,23 g: Saccharidpolymer (2)
- 2,50 g: Vinylacetat/Crotonsäure/Polyglykol Copolymer
- 0,20 g: Parfüm
- 0,07 g: 1-Amino-4-(2',3'-dehydroxypropyl)amino-5-chlor-2-nitrobenzol
- 0,05 g: Basic Brown 17 (C. I. 12 251)
- 0,01 g: Basic Blue 7 (C. I. 42 595)
- 0,0023g: Basic violett 14 (C. I. 42 510)
- 50,00 g: Ethanol
- ad 100 g: Wasser

### Beispiel 13: Dauerwellverformungsmittel

- 0,1 g: Saccharidpolymer (1)
- 8,0 g: Thioglykolsäure
- 2,6 g: Ammoniumhydrogencarbonat
- 0,3 g: Glycerin-polyethylenglykol-(35)-rizinoleat
- 0,3 g: Parfüm
- 0,1 g: Octylphenol, oxethyliert mit 20 Mol Ethylenoxid
- ad 100 g: Wasser

### Beispiel 14: Dauerwell-Fixiermittel

- 0,5 g: Saccharidpolymer (4)
- 10,0 g: Natriumbromat
- 3,2 g: Dinatriumhydrogenphosphat Dodecahydrat
- 0,8 g: ortho-Phosphorsäure (85prozentig)
- 0,5 g: Mononatriumphosphat
- ad 100 g: Wasser

### Beispiel 15: Schaumfixiermittel

- 0,2 g: Saccharidpolymer (2)
- 14,00 g: Wasserstoffperoxid (35prozentig)
- 3,41 g: o-Phosphorsäure (85prozentig)
- 1,25 g: DL-2-Pyrrolidon-5-carbonsäure
- 0,66 g: Laurylaminodimethylacetobetain
- 0,50 g: Polypropylen-(1)-polyethylen-(9)-laurylglykolether
- 0,20 g: Parfüm
- 0,05 g: p-Acetaminophenol
- ad 100 g: Wasser

## Patentansprüche

1. Haarbehandlungsmittel mit einem Gehalt an mindestens einem Polymer oder Copolymer, welches aus mindestens einer Monomerart aufgebaut ist, welche ausgewählt ist aus ethylenisch ungesättigten Sacchariden oder deren Derivaten und ethylenisch ungesättigten Saccharidsäuren oder deren Derivaten in einer geeigneten kosmetischen Grundlage, wobei die ungesättigten Saccharide und Saccharidsäuren in cyclischer oder offenkettiger Form vorliegen können.

2. Mittel nach Anspruch 1, dadurch gekennzeichnet, daß die ethylenische Doppelbindung der ungesättigten Saccharide oder Saccharidsäuren endocylisch, exocyclisch oder in der offenkettigen Form vorliegt.

3. Mittel nach einem der vorstehenden Ansprüche, dadurch gekennzeichnet, daß es sich bei den Derivaten der Saccharidsäuren um deren Salze, Ester, Lactone, Amide, Imide oder Nitrile handelt oder daß die Hydroxylgruppen organische Schutzgruppen tragen.

4. Mittel nach einem der vorstehenden Ansprüche, dadurch gekennzeichnet, daß es mindestens ein Monomer enthält, ausgewählt aus Verbindungen der allgemeinen Formeln I bis XVII: 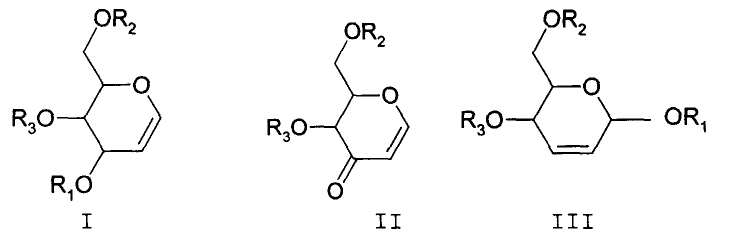 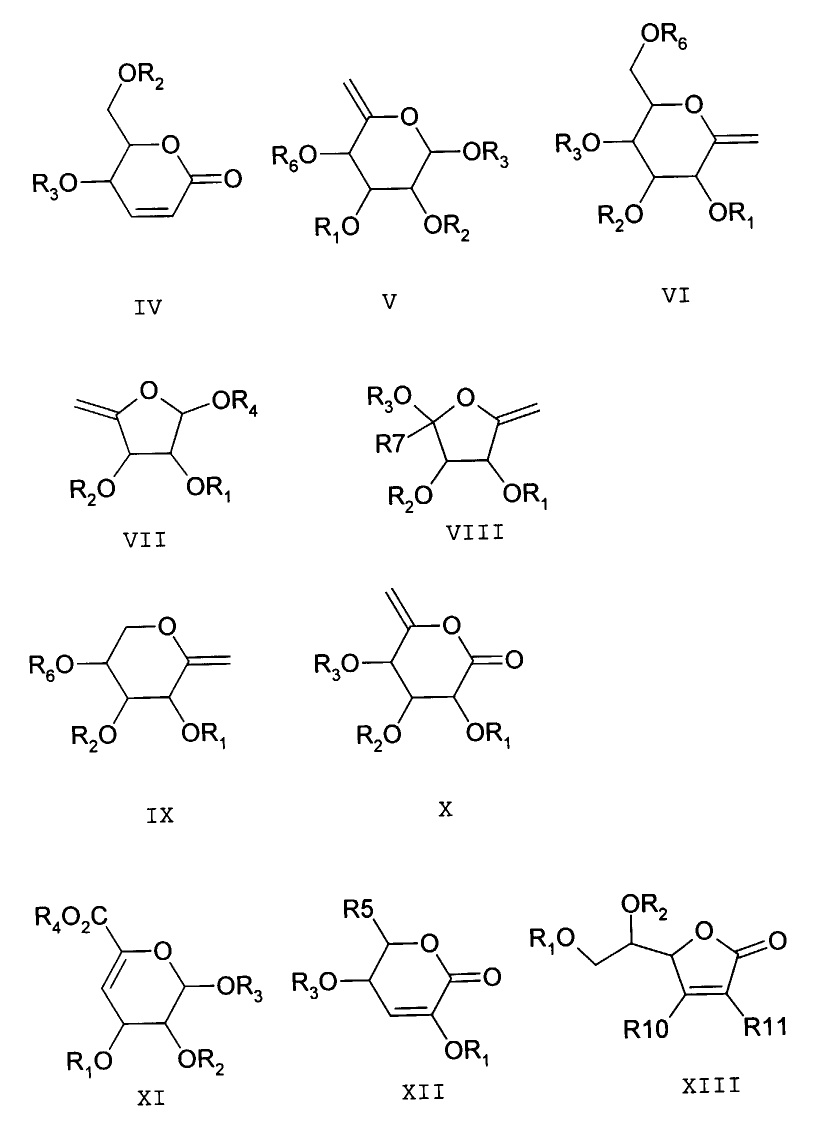 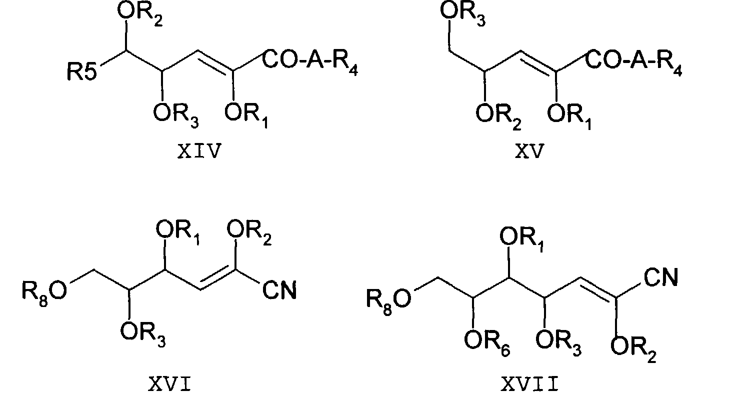 wobei R1, R2, R6 und R8 unabhängig voneinander für Wasserstoff, Alkyl, Aralkyl, Acyl oder Silyl stehen oder je zwei der genannten Reste bei geeigneter räumlicher Konfiguration Teil einer gemeinsamen Alkylidengruppe sind; R3 für einen der vorgenannten Reste oder für Glycosyl steht; R4 und R9 für Wasserstoff, Alkyl oder ein geeignetes Gegenion stehen; R5 für Wasserstoff, CH₂OR8 oder COOR9 steht; R7 für Wasserstoff oder O-Glycosyl steht; R10 und R11 unabhängig voneinander für Hydroxyl, Acetamido, O-Acyl oder O-Alkyl stehen, A für Sauerstoff, NH oder N-Alkyl steht und die genannten Alkylgruppen 1 bis 24 Kohlenstoffatome enthalten.

5. Mittel nach einem der vorstehenden Ansprüche, dadurch gekennzeichnet, daß das Polymer oder Copolymer zu 0,01 bis 25 Gew.% enthalten ist.

6. Mittel nach einem der vorstehenden Ansprüche, dadurch gekennzeichnet, daß es mindestens ein Copolymer enthält, welches aufgebaut aus ist aus mindestens einer Monomerart gemäß Anspruch 1 und mindestens einer weiteren, nicht von Sacchariden abgeleiteten, radikalisch polymerisierbaren Monomerart.

7. Mittel nach einem der vorstehenden Ansprüche, dadurch gekennzeichnet, daß es zusätzlich ein filmbildendes Polymer enthält.

8. Mittel nach Anspruch 7, dadurch gekennzeichnet, daß das zusätzliche filmbildende Polymer in einer Menge von 0,01 bis 25 Gew.% enthalten ist.

9. Verwendung von Polymeren oder Copolymeren, welche aus mindestens einer Monomerart aufgebaut sind, welche ausgewählt ist aus ethylenisch ungesättigten Sacchariden oder deren Derivaten und ethylenisch ungesättigten Saccharidsäuren oder deren Derivaten, wobei die ungesättigten Saccharide, Saccharidsäuren oder deren Derivate in cyclischer oder offenkettiger Form vorliegen können, zur Behandlung von Haaren.
